# EUROPEAN PATENT SPECIFICATION

(11) **EP 2 933 270 B1**
(45) Date of publication and mention of the grant of the patent: **06.09.2017**
(21) Application number: 13863004.1
(22) Date of filing: 10.12.2013
(51) Int. Cl.: C08B 37/16, A61K 31/366, A61P 35/00

(54) **ARTEANNUIN CYCLODEXTRIN CONJUGATE AND PREPARING METHOD THEREOF**
ARTEANNUIN-CYCLODEXTRIN-KONJUGAT UND HERSTELLUNGSVERFAHREN DAFÜR
CONJUGUÉ D'ARTÉANNUINE ET DE CYCLODEXTRINE ET SON PROCÉDÉ DE PRÉPARATION

(30) Priority: 14.12.2012 CN 201210544032
(43) Date of publication of application: 21.10.2015
(73) Proprietor: KPC Pharmaceuticals, Inc., Kunming, Yunnan 650106 (CN)
(72) Inventor: YANG, Bo, Kunming Yunnan 650106 (CN); CHEN, Yunjian, Kunming Yunnan 650106 (CN); YANG, Zhaoxiang, Kunming Yunnan 650106 (CN); ZHU, Ze, Kunming Yunnan 650106 (CN); XIAO, Dan, Kunming Yunnan 650106 (CN); ZHAO, Yulin, Kunming Yunnan 650106 (CN); LIAO, Xiali, Kunming Yunnan 650106 (CN)
(74) Representative: HGF Limited
(86) International application number: PCT/CN2013/088939
(87) International publication number: WO 2014/090128

(56) References cited:
- WO-A1-2004/075921
- WO-A1-2008/071851
- CN-A- 102 716 491
- US-A1- 2002 147 177
- NAKASE ET AL: "Anticancer properties of artemisinin derivatives and their targeted delivery by transferrin conjugation", INTERNATIONAL JOURNAL OF PHARMACEUTICS, ELSEVIER BV, NL, vol. 354, no. 1-2, 20 March 2008 (2008-03-20), pages 28-33, XP022550487, ISSN: 0378-5173, DOI: 10.1016/J.IJPHARM.2007.09.003
- BRUCE L. MAY ET AL: "Preparation and characterization of 6A-polyamine-mono-substituted [beta]-cyclodextrins", JOURNAL OF THE CHEMICAL SOCIETY, PERKIN TRANSACTIONS 1, vol. 1, no. 21, 1 January 1997 (1997-01-01), pages 3157-3160, XP055278917, GB ISSN: 0300-922X, DOI: 10.1039/a704467d

## Description

### TECHINICAL FIELD

This invention relates to the field of pharmaceutical synthesis, and in particular to an arteannuin-cyclodextrin conjugate and a preparation method thereof.

### BACKGROUND OF THE INVENTION

A new generation of antimalarial drug Artemisinin (sesquiterpene 1,2,4-trioxane (a sesquiterpene lactone endope roxide), Artemisinin, AMS) as a sesquiterpene lactone compound containing a peroxide group, which was isolated from Chinese herbal medicine *Artemisia annua L.* in 1970s, is an effective antimalarial drug against *Plasmodium falciparum* which has drug resistance to chloroquine. The arteannuin compounds, such as artemether, dihydroartemisinin, arteether and artesunate, are effective against *Plasmodium falciparum* resistant or sensitive to chloroquine and intracerebral malaria. The arteannuin combination therapy (ACT) recommended by the World Health Organization (WHO), which is rapid and reliable for people infected with malaria, is also widely accepted in a majority of countries where malaria is epidemic.

In recent years, researches indicate that the arteannuin compounds exhibit good effect for killing cancer cells. Arteannuin has the capability of selectively killing cancer cells with a minimum influence on normal cells. Unlike the action mechanisms of traditional chemotherapeutic drugs for tumor, arteannuin can reverse multidrug resistance of the tumor cells. The leukemic cell lines, which are resistant to doxorubicin, vincristine, methotrexate or hydroxyurea, have no cross resistance phenomenon to artesunate. The arteannuin drugs have already been used clinically as an antimalarial drug for many years, and thus safety thereof has been confirmed in thousands of clinical cases. However, antitumor mechanism thereof is still under study, which may be relevant to production of a large amount of free radicals due to reaction between arteannuin and Fe²⁺ and alkylation, and is also closely related to the induction of tumor cell apoptosis. The iron concentration in a cancer cell is much higher than that in a normal cell. The peroxide bridge in the structure of arteannuin is catalyzed by iron to split and produce free radicals, thereby killing the cells via a free radical pathway. In addition, researches demonstrate that arteannuin may induce the apoptosis of leukemic cancer cell in mice and hepatoma cells in human. However, the specific mechanism for stimulation (the drug acts on the target site) and effect (cell apoptosis) is still unclear. Researches further imply that arteannuin affects, to some extent, the tumor angiogenesis factors, and the artesunate has a broad-spectrum antitumor activity.

*In vitro* studies indicate that, the arteannuin drugs act on P388 leukemic cells cultured *in vitro,* and are capable of significantly inhibiting the proliferation of leukemic cells. Some arteannuic acids and arteannuic acid compounds selectively inhibit various cancer cells *in vitro.* Besides, sodium artesunate shows killing effect for human cervical cancer cell HeLa, shows killing effect for all human poorly differentiated squamous cell carcinoma of nasopharynx (HeLa, SUNE-1 and CNE2), significantly inhibits cell growth of hepatoma cell BEL-7402, and significantly inhibits cell growth of xenografted human hepatoma cell in nude mice. Artesunate induces the apoptosis of U937 cells, and has a killing effect on human colon cancer cell HCT-8, human erythroleukemia cell K562 and human breast cancer cell strain MCF-7.

In terms of aspect of *In vivo* experiments, experiments about the effect of different doses of artesunate on Hep2 hepatoma in mice shows that, intraperitoneal injection of artesunate at 60 mg/kg significantly inhibit mouse Hep2 hepatoma, with an anti-tumor rate of 80.4%. When influence of artesunate is studied by means of intramuscular injection, it is found that artesunate significantly inhibits both hepatoma in mice and solid tumor S180. As can be seen from the study on inhibition of artesunate on hepatocarcinoma H22 in mice, similar to positive control drug 5-Fu, when artesunate is administrated at 300 mg/kg by gavage, the anti-tumor rate is 40% or more. If a mouse tested is further administrated with dihydroartemisinin 6h after oral administration of ferrous sulfate, the growth of transplanted fibrosarcoma in the mouse may be significantly inhibited, and the effect thereof is greatly enhanced compared with the case in which dihydroartemisinin is used alone. In addition, arteannuin drugs may also inhibit growth of tumors such as human ovarian cancer HO-8910 transplanted tumor in nude mouse and prostate adenocarcinoma in a transgenic mouse.

However, the arteannuin compounds have low solubility in water, low bioavailability, and cannot be favorably transported onto cancer cells. In addition, they are eliminated rapidly in human and animal models, and thus cannot reach the therapeutic dosage at the surface of a cancer cell.

Cyclodextrins (CDs) are semi-natural occurring macromolecular compounds produced from starch by means of catalytic degradation of cyclodextrins glucosyltransferase. CDs are hardly hydrolyzable, and merely a small amount thereof is absorbed when passing through stomach and small intestine of a human body. However, CDs may be fermented by microorganisms in the colon and degraded into monosaccharide or disaccharide, and thus to be adsorbed by the large intestine. The present inventors have surprisingly found that, an arteannuin compound may be bonded with a cyclodextrin compound to produce a conjugate. The arteannuin-cyclodextrin conjugate can overcome the inherent defects of the arteannuin compounds, improve the therapeutic effect and reduce toxic or side effects.

### SUMMARY OF THE INVENTION

In view of this, the present invention aims to provide a targeting arteannuin-cyclodextrin conjugate, i.e., arteannuin compound - amine group modified cyclodextrin conjugate.

For the purpose of the present invention, the present invention adopts the following technical solutions:

an arteannuin-cyclodextrin conjugate, wherein an arteannuin compound is connected with a cyclodextrin modified by an amine group through an amide bond formed between a carboxyl group of the arteannuin compound and an amine group of the cyclodextrin modified by the amine group.

Those skilled in the art would appreciate that, "a conjugate" refers to a new molecule entity formed by connecting two or more molecules via covalent bonds. As used herein, the term "an arteannuin-cyclodextrin conjugate" refers to a compound formed by connecting an arteannuin compound with a cyclodextrin compound (a cyclodextrin modified by an amine group) via an amide bond, in particular, to a compound formed by an arteannuin compound in which the carboxyl group thereof forms an amide bond with an amine group of a cyclodextrin modified by the amine group.

The arteannuin compounds according to the present invention are those produced from substitution of C₁₂ of an arteannuin molecule with a compound comprising a carboxyl group. Synthesis of the arteannuin compounds may be carried out as described in the documents of the prior art.

Cyclodextrin (CD) is a generic term for a series of cyclic oligosaccharide produced by amylose with the action of cyclodextrin glucosyltransferase generated by Bacillus cereus. Among which, molecules containing 6, 7 or 8 glucose units (which are known as α-, β-, and γ-cyclodextrin, respectively), attract much more attention and are of great practical value. Based on results from X-ray crystal diffraction, infrared spectrum and nuclear magnetic resonance spectroscopy, it is determined that each D(+)-glucopyranose constituting cyclodextrin molecule has chair conformation, and each glucose unit is connected with 1,4-glycosidic bond to form a ring. Cyclodextrin has a hollow cylindric steric loop structure with two open ends in which one end is big and the other end is small, since the glycosidic bond connecting the glucose units cannot rotate freely. In the hollow structure thereof, a hydrophobic region is formed due to a shielding effect caused by the C-H bond, while all the hydroxyl groups locate at the external of the molecule. The end with large opening is composed of C₂ and C₃ secondary hydroxyl group, and the end with small opening is composed of C₆ primary hydroxyl group, which is highly hydrophilic, and the cyclodextrin has the structure of: 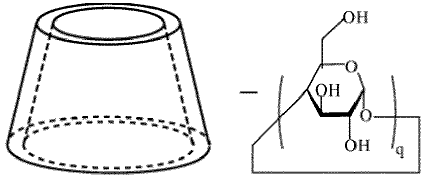 wherein the compound is α-cyclodextrin when q=6, β-cyclodextrin when q=7, and γ-cyclodextrin when q=8.

The cyclodextrin modified by an amine group is a compound produced by substitution of hydroxyl group of C₂, C₃ and/or C₆ of D(+)-glucopyranose constituting the cyclodextrin molecule with an amine group. Synthesis of the cyclodextrin modified by an amine group may be carried out as described in the prior art documents. The cyclodextrin may firstly be reacted with a sulfonylating reagent to produce a sulfonylated cyclodextrin [see R.C. Petter, J.S. Salek, C.T. Sikorski, G. Kumaravel, and F.-T. Lin: J. Am. Chem. Soc. 112, 3860-3868(1990)], and the cyclodextrin may be sulfonylated at 2-, 3- and/or 6-position of the D(+)-glucopyranose. The sulfonylating reagent commonly used is benzene sulfonyl chloride and *p*-methyl benzene sulfonyl chloride. Then, under the nucleophilic attack of the aminating agent, the sulfonyl group of the sulfonated cyclodextrin leaves and is substituted with an amine group, thereby to produce the cyclodextrin modified by an amine group [see B.L. May, S.D. Kean, C.J. Easton, and S.F. Lincoln: J. Chem. Soc., Perkin Trans. 13157-3160 (1997)]. The aminating agent may be various organic reagents containing an amine group, including ammonia, methylamine, ethylamine, propylamine, butylamine, ethylenediamine, ethanolamine, acetamide and diethylene triamine. For example, *p*-toluenesulfonyl chloride is reacted with β-cyclodextrin to produce 6-*p*-toluenesulfonyl-β-cyclodextrin (6-OTs-β-CD). Then, 6-OTs-β-CD is added to an ethylenediamine solution, thereby to obtain ethylenediamine modified β-cyclodextrin. The reaction equation is as follows:

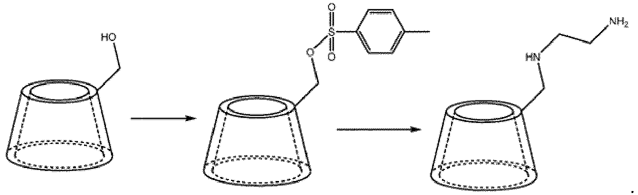

Preferably, the cyclodextrin modified by an amine group has a structure shown in formula I: 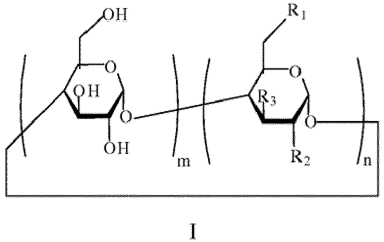
wherein m is 0 to 7; n is 1 to 8, and m+n=any one of 6, 7 and 8;
R₁, R₂ and R₃ represent -OH or -RNH₂, and at least one of R₁, R₂ and R₃ represents -RNH₂;
R is (CH₂)_{X}, NH(CH₂)_{X}, NH(CH₂)_{X}NH(CH₂)_{X}, CO(CH₂)_{X} or O(CH₂)_{X}, and x is an integer greater than or equal to 0.

For formula I, the term "m+n=any one of 6, 7 or 8" means that, the cyclodextrin modified by an amine group according to the present invention may be α-, β- or γ-cyclodextrin; the term "n is at least 1" means that at least one D(+)-glucopyranose among the cyclodextrin molecule modified by an amine group is modified by an amine group, whereby m is 5, 6 or 7; and the term "m is 0" means the cyclodextrin modified by an amine group, in which each D(+)-glucopyranose constituting the cyclodextrin molecule is modified by an amine group.

The term "at least one of R₁, R₂ and R₃ in formula I represents -RNH₂" means the cyclodextrin modified by the amine group, wherein the amine group modification of D(+)-glucopyranose molecule is at least monoamine modification which may occur at 2-, 3- or 6-position, or may be diamine modification; alternatively, R₁, R₂ and R₃ are all modified.

R in the amine group -RNH₂ for modifying cyclodextrin is also defined in formula I. The term "R represents (CH₂)_{X}, NH(CH₂)_{X}, NH(CH₂)_{X}NH(CH₂)_{X}, CO(CH₂)_{X} or O(CH₂)_{X}" means that, the amine group for modifying cyclodextrin may be an organic amine group, such as ammonia, methylamine, ethylamine, ethylenediamine, ethanolamine, acetamide and diethylene triamine, wherein x is an integer greater than or equal to 0, preferably 0 to 10, more preferably 0, 1, 2, 3 or 4.

Preferably, the cyclodextrin modified by an amine group has a structure shown in formula I: 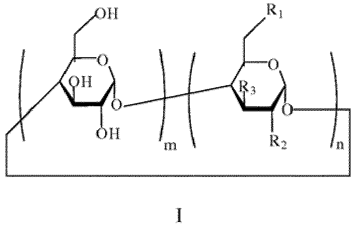
wherein n is 1, and m+n= any one of 6, 7 and 8;
R₁, R₂ or R₃ is -RNH₂;
R is (CH₂)_{X}, NH(CH₂)_{X}, NH(CH₂)_{X}NH(CH₂)_{X}, CO(CH₂)_{X} or O(CH₂)_{X}, and x is 0, 1, 2, 3 or 4.

More preferably, the cyclodextrin modified by an amine group is selected from the group consisting of mono-[6-(ethanediamine)-6-deoxy]-β-cyclodextrin, mono-[2-(ethanediamine)-6-deoxy]-β-cyclodextrin, mono-[3-(methylamino)-6-deoxy]-α-cyclodextrin, and mono-[6-(ethanolamine)-6-deoxy]- γ-cyclodextrin.

The arteannuin compounds according to the present invention are those produced by substitution of C₁₂ constituting the arteannuin molecule with a compound containing a carboxyl group. The synthesis of the arteannuin compounds may be carried out as descried in the documents of the prior art.

The dihydroarteannuin may be reacted with the compound containing a carboxyl group to produce an arteannuin compound [see P. M. O' Neill, et al.: J. Med. Chem. 44, 58-68(2001)]. The dihydrarteannuin may be etherified or esterified at hydroxyl group of C₁₂, in which the etherifying or esterifying reagent may be various compounds containing organic groups of a carboxyl group and a hydroxyl group, and the reaction equation is as follows:

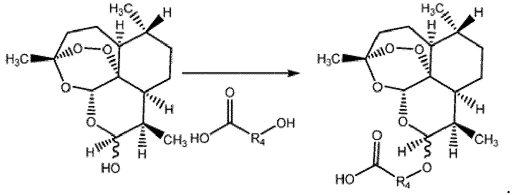

Preferably, the cyclodextrin modified by an amine group has a structure shown in formula II: 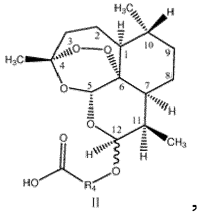 wherein R₄ is (CH₂)_{y}, CO(CH₂)_{y}, C₆H₆(CH₂)_{y} or COC₆H₆(CH₂)_{y}; y is an integer greater than or equal to 0; CO is carbonyl; and C₆H₆ is a benzene ring.

More preferably, the etherifying or esterifying reagent is succinic acid, succinic anhydride, terephthalic acid, *p*-hydroxybenzoic acid, *p*-hydroxybenzoate, *p*-halobenzoic acid and *p*-halobenzoic acid.

More preferably, the arteannuin compound is artesunate, a compound in which C-12 of the arteannuin is connected with a phenoxy group and a compound in which C-12 of the arteannuin is connected with an alkoxy group.

Another object of the present invention is to provide a method for preparing the arteannuin-cyclodextrin conjugate according to the present invention.

A method for preparing the arteannuin-cyclodextrin conjugate according to the present invention comprises: in the presence of a condensation agent, carrying out an amidation reaction between a carboxyl group of the arteannuin compound and an amine group of a cyclodextrin modified by the amine group in an organic solvent having a strong polarity, thereby to obtain the arteannuin-cyclodextrin conjugate.

The condensation agent useful for the preparation method of the present invention comprises various condensation agents used in the amidation reaction in the pharmaceutical synthesis, including carbodiimide condensation agents and onium salt condensing reagents, in which the carbodiimide condensation agents are widely used in the amide preparation of the pharmaceutical synthesis. The condensation agent used in the preparation method according to the present invention is preferably carbodiimide condensation agents.

In the preparation method according to the present invention, there are mainly three carbodiimide condensation agents: dicyclohexyl carbodiimide (DCC), diisopropyl carbodiimide (DIC) and 1-(3-dimethylamino-propyl)-3-ethylcarbodiimide (EDCI). Preferred condensation agents in the present invention are complex condensation agent of dicyclohexyl carbodiimide and 1-hydroxy benzotriazole (DCC-HOBt).

Preferably, the weight ratio of the cyclodextrin modified by an amine group to the dicyclohexyl carbodiimide is 1:0.04∼10, more preferably, 1:0.4∼5.

Preferably, the weight ratio of the cyclodextrin modified by an amine group to the 1-hydroxylbenzotriazole is 1:0.03∼7.5, more preferably, 1:0.3∼3.

More preferably, a dehydrating reagent may also be used in the amidation reaction according to the present invention, so as to absorb the water molecules produced by the condensation reaction, thereby to prevent the condensation product from being hydrolyzed and to improve efficiency of the condensation reaction. The dehydrating reagent includes calcium oxide, phosphorous pentoxide, molecular sieve and the like.

The cyclodextrin modified by an amine group is soluble in water and an organic solvent having strong polarity, while the arteannuin compound, dicyclohexyl carbodiimide and 1-hydroxylbenzotriazole are all insoluble in water. Moreover, the amidation between the carboxyl group and the amine group is a dehydrating condensation reaction. The amidation is disadvantageous when water is used as a solvent. Therefore, the preparation method according to the present invention uses an organic solvent having strong polarity as a solvent.

Preferably, the organic solvent having strong polarity is N,N-dimethylamide, N,N-diethylamide, or dimethyl sulfoxide.

Preferably, the amidation reaction in the preparation method according to the present invention comprises: reacting the cyclodextrin modified by an amine group with excess arteannuin compound under the condition of -10°C to 10°C for 6 h to 24 h to form a carboxyl group-activated intermediate, and then carrying out an amidation reaction between the carboxyl group of artesunate and the amine group of the cyclodextrin under the condition of 15°C to 40°C for 6 to 24 h, thereby to produce the arteannuin-cyclodextrin conjugate.

The preparation method according to the present invention further comprises a step of activating the surface hydroxyl group of the cyclodextrin modified by an amine group before the amidation reaction, which comprises reacting the cyclodextrin modified by an amine group with a condensation agent under the condition of -10°C to 10°C for 0.5 h to 5 h.

The preparation method according to the present invention further comprises a step of purifying the arteannuin-cyclodextrin conjugate.

Since dicyclohexyl carbodiimide and 1-hydroxylbenzotriazole are insoluble in water, the arteannuin compound has poor water solubility, and is hardly soluble in water. However, for the arteannuin-cyclodextrin conjugate according to the present invention, the arteannuin compound is connected with the cyclodextrin modified by an amine group via an amide bond. Thus, the arteannuin-cyclodextrin conjugate according to the present invention has many hydrophilic active groups, thus has better water solubility and dissolves easily in water. The purifying step according to the present invention may comprises drying the resultant solution obtained from the amidation reaction, collecting the solid residues and dissolving it in water, then filtrating it to remove contaminants, and isolating by a method of organic solvent precipitation, thereby obtaining the arteannuin-cyclodextrin conjugate in its pure form. The filtrating to remove contaminant aims to remove unreacted artesunate, dicyclohexyl carbodiimide and 1-hydroxylbenzotriazole.

Without wishing to be limited by any theory, the principle for preparing the arteannuin-cyclodextrin conjugate by separation through organic solvent precipitation involves that, the organic solvent can reduce the dielectric constant of the solution, and reduce the polarity of the solvent, thereby to decrease the interaction between solvent molecules and the arteannuin-cyclodextrin conjugate molecules, and the arteannuin-cyclodextrin conjugate is thus precipitated out due to decrease in solubility.

Preferably, the organic solvent is acetone, methanol, ethanol, isopropanol, chloroform or tetrahydrofuran.

The organic solvent precipitation method according to the present invention is affected by the concentration of the solution. In case of the solution with a low concentration, the recovery rate is low since the arteannuin-cyclodextrin conjugate is not sufficiently precipitated out. As such, a larger proportion of organic solvent is needed to perform precipitation. The organic solvent may be saved when a solution with a high concentration is used. The preparation method according to the present invention can further comprise a step of concentrating in order to reduce water in the solution, improve concentration of the solution, thereby to fully precipitate the arteannuin-cyclodextrin conjugate out from the organic solvent.

The preparation method according to the present invention further comprises a step of refining the arteannuin-cyclodextrin conjugate.

Preferably, the refining is an organic solvent extraction or chromatography.

The organic solvent is selected from the group consisting of acetone, diethyl ether, chloroform and tetrahydrofuran.

In a particular embodiment, the structure of the arteannuin-cyclodextrin conjugate is determined by NMR and high-resolution mass spectrometry in the present invention. The ¹HNMR graph of the arteannuin-cyclodextrin conjugate shows that, under the condition of D₂O H₅, H₁₂, H₁₃, H₁₄ and H₁₅ characteristic peaks of the arteannuin compounds occur at 0.3 to 3.0 ppm and 5.0 to 7.1 ppm, where cyclodextrin does not have a characteristic absorption hereby. In addition, artesunate is almost insoluble in water, which can initially indicate that artesunate already reacts with cyclodextrin. The ¹³CNMR graph of the arteannuin-cyclodextrin conjugate shows that, C backbone characteristic peak of the arteannuin occurs at 10 to 50ppm, and C backbone characteristic peak of the carbonyl in the arteannuin compound occurs at 170 to 190 ppm, which indicates that amidation reaction occurs at the carboxyl group of the artesunate. It shows that, the artesunate is bonded to the cyclodextrin via condensation between O=C-OH and -NH₂. High-resolution mass spectrum shows that *m*/*z*: 1543.5715(M⁺).

The arteannuin-cyclodextrin conjugate according to the present invention uses a cyclodextrin as a carrier, and a molecule of an arteannuin compound is connected, via an amide bond formed between the carboxyl group thereof and any amine group of the cyclodextrin modified by an amine group, to the cyclodextrin. Compared with arteannuin and dihydroartemisinin, the arteannuin-cyclodextrin conjugate according to the present invention has more hydrophilic active groups. It has good biocompatibility and better water solubility than arteannuin and dihydroartemisinin. The arteannuin-cyclodextrin conjugate according to the present invention has a solubility of 45 to 98 mg/mL in water under 25°C, while arteannuin and dihydroarteannuin are almost insoluble in water.

In another aspect, the arteannuin-cyclodextrin conjugate according to the present invention has anticancer targeting feature, especially for target site of colorectal cancer cells. Only a mall amount of the arteannuin-cyclodextrin conjugate is absorbed when passing through stomach and small intestine of a human body, and it merely decomposes and releases in the colon, and then is absorbed by the large intestine, and thus has the feature of releasing in the colon. The arteannuin-cyclodextrin conjugate according to the present invention has anticancer targeting feature, especially for colorectal cancer cells, which can effectively enter the body of a patient, gather around targeting cancer cells preferentially, and selectively induce rectum cancer cells in a human body to die, thereby improving pharmacodynamic effects and reducing toxic or side effects.

For example, 180 µl suspension of human rectal cancer cells HCT116, Lovo, SW480 and HT29 at a concentration of 4×10⁴/mL is added to 96-well culture plate, and pre-cultured under the conditions of 5% CO₂, 37°C and saturated moisture for 24h. After 150 µl medium is replaced, 20 µl medium containing molecular assembly at different concentrations is added, and cultured for another 48 h, with the medium free of a sample as a negative control, and a medium containing camptothecin, oxaliplatin and fluorouracil at different concentrations as a positive control. MTT assay is used to determine the OD value, and to calculate the growth inhibition rate. The inhibition of compounds at high, medium and low concentrations on the tumor cell proliferation is observed. All the experiments above are performed in triplet. Results for *in vitro* experiment of the arteannuin-cyclodextrin conjugate with respect to human rectum and caecum cancer cells are shown in table 1.

**Table 1 IC₅₀ value (µM) for certain arteannuin compounds in several rectum cancer cells**

| (HCT116, Lovo, SW480 and HT90 Cells) | | | | | |
|---|---|---|---|---|---|
| compounds | MW | IC₅₀(µM) | | | |
| | | HCT116 | Lovo | SW480 | HT 90 |
| oxaliplatin | 397 | 0.76 | 1.51 | 30.1 | 9.7 |
| fluorouracil | 130 | 5.4 | 23.0 | 30.8 | 38.5 |
| dihydroarteannuin | 284 | 1.76 | 2.11 | 14.1 | 21.1 |
| artesunate | 384 | 1.04 | 2.60 | 1.82 | 9.11 |
| mono-[6-(ethanediamine)]-6 -deoxy]-β-cyclodextrin -artesunate conjugate | 1543 | 0.58 | 1.62 | 3.89 | 5.18 |

As can be seen from table 1, an arteannuin-cyclodextrin conjugate with suitable linkage length (such as, mono-[6-(ethanediamine)-6-deoxy]-β-cyclodextrin-artesunate conjugate) is designed and synthesized, which have an anticancer activity better than most of the reaction raw materials (dihydroarteannuin and artesunate) and medicines used clinically (oxaliplatin and fluorouracil). The reasons probably lie in that, the linkage with suitable length for linking cyclodextrin and artesunate can gather around the targeting rectum and caecum cancer cells preferentially and selectively induce rectum cancer cells in a human body to die. In view of the fact that cyclodextrin can be decomposed into open chain maltodextrin, maltose and glucose by microbial community in the colon tract, and then absorbed by the large intestine, it can be used as a carrier of drugs at colorectal target site for transporting the drugs to rectum, thereby improving the bioavailability of the drugs at the colon and exerting the function of the drugs. Therefore, the arteannuin-cyclodextrin conjugate according to the present invention has anticancer targeting feature, especially for colorectal cancer cells, which can effectively enter the body of a patient, gather around the targeting cancer cells preferentially, and selectively induce rectum cancer cells in a human body to die, thereby improving pharmacodynamic effects and reducing toxic or side effects.

The method for preparing the arteannuin-cyclodextrin conjugate in the present invention is characterized by simple operations, easy availability of raw materials and moderate reaction conditions, and can be used for large scale preparation of the arteannuin-cyclodextrin conjugate.

### BRIEF DESCRIPTION OF THE DRAWINGS

FIG. 1 shows a diagram for ¹HNMR of β-cyclodextrin.
FIG.2 shows a diagram for ¹HNMR of mono-[2-(ethanediamine)-6-deoxy]-β-cyclodextrin-artesunate conjugate prepared in example 1, with H₅, H₁₂, H₁₃, H₁₄ and H₁₅ characteristic peaks at 7 to 8 ppm; however, cyclodextrin does not have characteristic peaks hereby.
FIG.3 shows a diagram for ¹³CNMR of mono-[2-(ethanediamine)-6-deoxy]-β-cyclodextrin-artesunate conjugate prepared in example 1, with unsaturated C backbone absorption characteristic peaks of artesunate at 10 to 50 ppm; and unsaturated carbonyl adsorption peak of amide bond at 179 to 190 ppm;
FIG.4 shows a high-resolution mass spectrum diagram [HSMS(TOF-ESI)] of the mono-[2-(ethanediamine)-6-deoxy]-β-cyclodextrin-artesunate conjugate prepared in example 1.

### DETAILED DESCRIPTION OF EMBODIMENTS

The examples of the present invention disclose the arteannuin-cyclodextrin conjugates and preparation method thereof. Those skilled in the art may modify the parameters in the process according to the disclosure of the present invention. It is to be noted that, all the similar replacements and modification are apparent for those skilled in the art, and thus are contemplated in the present invention. The products and methods of the present invention have been described by preferred embodiments. It is apparent that those skilled in the art can make various modifications to the product and method of the present invention or suitable changes and combination thereof to achieve and apply the technology of the present invention without departing from the content, spirit and scope of the invention.

The present invention will be illustrated in detail in combination with examples below in order to further understand the present invention.

### Example 1:

### 1. Preparation of sulfonylated cyclodextrin

With reference to the document [R.C. Petter, J.S. Salek, C.T. Sikorski, G. Kumaravel, and F. -T. Lin: J. Am. Chem. Soc. 112, 3860-3868(1990)], recrystallized β-cyclodextrin (210 g) was dissolved in distilled water (1300 mL), a white emulsion was obtained after the solution was fully stirred. A sodium hydroxide solution (17.2 g, 50 mL) was added, and stirred for 1.5 h. Then, *p*-toluensulfonyl chloride (26.0 g) was weighed and dissolved in an acetonitrile solution (80 ml). The resultant solution was added dropwise to β-cyclodextrin basic solution, and stirred at room temperature for 2h. A small amount of insoluble matters were filtered by suction, and the pH of the filtrate was adjusted to 7.5 with 2M HCl. At the same time, a large amount of precipitate was produced, and the filtrate was filtered by suction. The precipitate was then dissolved in water (450 mL) under heating, and insoluble matters were filtered out as it was hot. The filtrate was recrystallized for 12 h under 0°C. The precipitate obtained after filtration was then recrystallized for multiple times with hot water. The resultant was dried at 60°C under vacuum for 12 h, thereby to obtain mono-6-*p*-tolubene sulfonyl-β-cyclodextrin in a pure form (about 18 g, yield 8%).

### 2. Preparation of mono-[6-(ethanediamine)-6-deoxy]-β-cyclodextrin

With reference to the document [B.L. May, S.D. Kean, C.J. Easton, and S.F. Lincoln: J. Chem. Soc., Perkin Trans. 13157-3160(1997)], mono-6-*p*-tolubene sulfonyl-β-cyclodextrin (3 g) was added to ethylenediamine solution (20 mL), and reacted under 80°C for 8 h. After cooling, the resultant solution was dropped into acetone. Then, the precipitate was collected, thereby to obtain mono-[6-(ethanediamine)-6-deoxy]-β-cyclodextrin (2.3g, yield 84%).

### 3. Preparation of mono-[6-(ethanediamine)-6-deoxy]-β-cyclodextrin-artesunate conjugate

Mono-[2-(ethanediamine)-6-deoxy]-β-cyclodextrin (2.61 g, 2 mmol) was dissolved in anhydrous N,N-dimethylamide (50 mL), and cooled to about 0°C. To this solution, dicyclohexyl carbodiimide (DCC, 1.2 g) and 1-hydroxylbenzotriazole (HOBT, 0.9g) were added, and stirred for 5 h in an ice bath. Then, artesunate (2.77g, 6 mmol) was added. The reaction solution was stirred for 20 h under 0°C, and then stirred for 10 h under 25°C (TCL tracking detection, and the developer is methanol: ethyl acetate: water=7:7:1). Then, the reaction solution was evaporated to dryness under reduced pressure at 60°C. The residue was fully dissolved in water, filtered, concentrated in the filtrate, dropped in chloroform (100 mL) for extraction and filtered. The precipitate was collected and dried for 24 h under vacuum at 50°C, thereby to obtain mono-[6-(ethanediamine)-6-deoxy]-β-cyclodextrin-artesunate conjugate (yield: 65%; solubility: 68 mg/mL).

The results for NMR and high-resolution mass spectrum are shown in FIGs. 2, 3 and 4. The ¹HNMR graph of the arteannuin-cyclodextrin conjugate shows that, under the condition of D₂O, H₅, H₁₂, H₁₃, H₁₄ and H₁₅ characteristic peaks of the arteannuin compounds occur at 0.3 to 3.0 ppm and 5.0 to 7.1 ppm, while cyclodextrin does not have a characteristic absorption hereby. In addition, the artesunate is almost insoluble in water, which can preliminarily indicate that the artesunate already reacts with the cyclodextrin. The ¹³CNMR graph of the arteannuin-cyclodextrin conjugate shows that, C backbone characteristic peak of the arteannuin occurs at 10 to 50 ppm, and C backbone characteristic peak of carbonyl in the arteannuin compound occurs at 170 to 190 ppm, which indicates that an amidation reaction occurs at the carboxyl group of the artesunate. It shows that, the artesunate is bonded to cyclodextrin through condensation between O=C-OH and -NH₂. High-resolution mass spectrum shows that *m*/*z:* 1543.5715(M⁺).

### Example 2

Mono-[6-(monoamine)-6-deoxy]-β-cyclodextrin (2.61 g, 2 mmol) was dissolved in anhydrous N,N-diethylamide (50 mL), and cooled to about -10°C. To this solution, dicyclohexyl carbodiimide (DCC, 1.2 g) was added, and stirred for 0.5 h in an ice bath. Then, artesunate (2.77g, 6 mmol) was added. The reaction solution was stirred for 6 h under 10°C, and then stirred for 4 h under 45°C. Then, the reaction solution was evaporated to dryness under reduced pressure at 60°C. The residue was fully dissolved in water, filtered, concentrated in the filtrate, dropped in tetrahydrofuran (100 mL) for extraction and filtered. The precipitate was collected and dried for 24 h under vacuum at 50°C, thereby to obtain mono-[6-(monoamine)-6-deoxy]-β-cyclodextrin-artesunate conjugate (yield: 58%; solubility: 45 mg/mL).

### Example 3

Mono-[3-(diethylenetriamine)-6-deoxy]-α-cyclodextrin (2 mmol) was dissolved in anhydrous dimethyl sulfoxide (50 mL), and cooled to about -5°C. To this solution, dicyclohexyl carbodiimide (DCC, 1.2 g) and 1-hydroxylbenzotriazole (HOBT, 0.9g) were added, and stirred for 2.5 h in an ice bath. Then, artesunate (2.77g, 6 mmol) was added. The reaction solution was stirred for 12 h under 0°C, and then stirred for 24 h under 25°C. Then, the reaction solution was evaporated to dryness under reduced pressure at 60°C. The residue was fully dissolved in water, filtered, concentrated in the filtrate, dropped in isopropanol (100 mL) and filtered. The precipitate was collected and dried for 24 h under vacuum at 50°C, thereby to obtain mono-[3-(diethylenetriamine)-6-deoxy]-α-cyclodextrin-artesunate conjugate (yield: 56%; solubility: 78 mg/mL).

### Example 4

Mono-[6-(ethanolamine)-6-deoxy]-γ-cyclodextrin (1 mmol) was dissolved in anhydrous N,N-dimethylamide (50 mL), and cooled to about 0°C. To this solution, dicyclohexyl carbodiimide (DCC, 1.2 g) was added, and stirred for 24 h in an ice bath. Then, artesunate (2.77g, 6 mmol) was added. The reaction solution was stirred for 24 h under 10°C, and then stirred for 12 h under 45°C. Then, the reaction solution was evaporated to dryness under reduced pressure at 60°C. The residue was fully dissolved in water and filtered. The filtrate was concentrated, and column chromatography using water as an eluent was performed. The solution obtained from the chromatography was collected and concentrated, then dried for 24 h under vacuum at 50°C, thereby to obtain mono-[6-(ethanolamine)-6-deoxy]-γ-cyclodextrin-artesunate conjugate (yield: 57%; solubility: 75 mg/mL).

### Example 5

Mono-[2-(ethanediamine)-6-deoxy]-β-cyclodextrin (2.61 g, 2 mmol) was dissolved in anhydrous N,N-dimethylamide (50 mL), and cooled to about 0°C. To this solution, dicyclohexyl carbodiimide (DCC, 1.2 g) and 1-hydroxylbenzotriazole (HOBT, 0.9g) were added, and stirred for 5 h in an ice bath. Then, a compound in which C-12 of the arteannuin was connected with a phenoxy group (3.77g, 6 mmol) was added. The reaction solution was stirred for 20 h under 0°C, and then stirred for 10 h under 25°C (TCL tracking detection was adopted, and the developer was methanol: ethyl acetate: water=7:7:1). Then, the reaction solution was evaporated to dryness under reduced pressure at 60°C. The residue was fully dissolved in water, filtered, concentrated in the filtrate, dropped in chloroform (100 mL) for extraction and filtered. The precipitate was collected and dried for 24 h under vacuum at 50°C, thereby to obtain a conjugate of mono-[6-(ethanediamine)-6-deoxy]-β-cyclodextrin and the compound in which C-12 of the arteannuin was connected with a phenoxy group (yield: 65%; solubility: 68 mg/mL).

### Example 6

Mono-[6-(monoamine)-6-deoxy]-β-cyclodextrin (2.61g, 2 mmol) was dissolved in anhydrous N,N-diethylamide (50 mL), and cooled to about -10°C. To this solution, dicyclohexyl carbodiimide (DCC, 1.2 g) was added, and stirred for 0.5 h in an ice bath. Then, a compound in which C-12 of the arteannuin was connected with a phenoxy group (3.77g, 6 mmol) was added. The reaction solution was stirred for 6 h under 10°C, and then stirred for 4 h under 45°C. Then, the reaction solution was evaporated to dryness under reduced pressure at 60°C. The residue was fully dissolved in water, filtered, concentrated in the filtrate, dropped in tetrahydrofuran (100 mL) for extraction and filtered. The precipitate was collected and dried for 24 h under vacuum at 50°C, thereby to obtain a conjugate of mono-[6-(monoamine)-6-deoxy]-β-cyclodextrin and the compound in which C-12 of the arteannuin was connected with a phenoxy group (yield: 58%; solubility: 54 mg/mL).

### Example 7

Mono-[3-(diethylenetriamine)-6-deoxy]-α-cyclodextrin (2 mmol) was dissolved in anhydrous dimethyl sulfoxide (50 mL), and cooled to about -5°C. To this solution, dicyclohexyl carbodiimide (DCC, 1.2 g) and 1-hydroxylbenzotriazole (HOBT, 0.9g) were added, and stirred for 2.5h in an ice bath. Then, a compound in which C-12 of the arteannuin was connected with a phenoxy group (3.77g, 6 mmol) was added. The reaction solution was stirred for 12 h under 0°C, and then stirred for 24 h under 25°C. Then, the reaction solution was evaporated to dryness under reduced pressure at 60°C. The residue was fully dissolved in water, filtered, concentrated in the filtrate, dropped in isopropanol (100 mL) and filtered. The precipitate was collected and dried for 24 h under vacuum at 50°C, thereby to obtain a conjugate of mono-[3-(diethylenetriamine)-6-deoxy]-α-cyclodextrin and the compound in which C-12 of the arteannuin was connected with a phenoxy group (yield: 56%; solubility: 88 mg/mL).

### Example 8

Mono-[6-(triethylenetetraamine)-6-deoxy]-γ-cyclodextrin (1 mmol) was dissolved in anhydrous dimethylamide (50 mL), and cooled to about 0°C. To this solution, dicyclohexyl carbodiimide (DCC, 1.2 g) was added, and stirred for 24 h in an ice bath. Then, a compound in which C-12 of the arteannuin was connected with a phenoxy group (3.77g, 6 mmol) was added. The reaction solution was stirred for 24 h under 10°C, and then stirred for 12 h under 45°C. Then, the reaction solution was evaporated to dryness under reduced pressure at 60°C. The residue was fully dissolved in water and filtered. The filtrate was concentrated, and column chromatography using water as an eluent was performed. The solution obtained from the chromatography was collected and concentrated, then dried for 24 h under vacuum at 50°C, thereby to obtain a conjugate of mono-[6-(triethylenetetraamine)-6-deoxy]-γ-cyclodextrin and the compound in which C-12 of the arteannuin was connected with a phenoxy group (yield: 57%; solubility: 95 mg/mL).

### Example 9

Mono-[2-(ethanediamine)-6-deoxy]-β-cyclodextrin (2.61 g, 2 mmol) was dissolved in anhydrous N,N-dimethylamide (50 mL), and cooled to about 0°C. To this solution, dicyclohexyl carbodiimide (DCC, 1.2 g) and 1-hydroxylbenzotriazole (HOBT, 0.9g) were added, and stirred for 5 h in an ice bath. Then, a compound in which C-12 of the arteannuin was connected with an alkoxy group (3.47g, 6 mmol) was added. The reaction solution was stirred for 20 h under 0°C, and then stirred for 10 h under 25°C (TCL tracking detection was adopted, and the developer is methanol: ethyl acetate: water=7:7: 1). Then, the reaction solution was evaporated to dryness under reduced pressure at 60°C. The residue was fully dissolved in water, filtered, concentrated in the filtrate, dropped in chloroform (100 mL) for extraction and filtered. The precipitate was collected and dried for 24 h under vacuum at 50°C, thereby to obtain a conjugate of mono-[6-(ethanediamine)-6-deoxy]-β-cyclodextrin and the compound in which C-12 of the arteannuin was connected with an alkoxy group (yield: 65%; solubility: 71 mg/mL).

### Example 10

Mono-[6-(monoamine)-6-deoxy]-β-cyclodextrin (2.61 g, 2 mmol) was dissolved in anhydrous N,N-diethylamide (50 mL), and cooled to about -10°C. To this solution, dicyclohexyl carbodiimide (DCC, 1.2 g) was added, and stirred for 0.5 h in an ice bath. Then, a compound in which C-12 of the arteannuin was connected with an alkoxy group (3.47g, 6 mmol) was added. The reaction solution was stirred for 6 h under 10°C, and then stirred for 4 h under 45°C. Then, the reaction solution was evaporated to dryness under reduced pressure at 60°C. The residue was fully dissolved in water, filtered, concentrated in the filtrate, dropped in teterhydrofuran (100 mL) for extraction and filtered. The precipitate was collected and dried for 24 h under vacuum at 50°C, thereby to obtain a conjugate of mono-[6-(monoamine)-6-deoxy]-β-cyclodextrin and the compound in which C-12 of the arteannuin was connected with an alkoxy group (yield: 58%; solubility: 52 mg/mL).

### Example 11

Mono-[3-(diethylenetriamine)-6-deoxy]-α-cyclodextrin (2 mmol) was dissolved in anhydrous dimethyl sulfoxide (50 mL), and cooled to about -5°C. To this solution, dicyclohexyl carbodiimide (DCC, 1.2 g) and 1-hydroxylbenzotriazole (HOBT, 0.9g) were added, and stirred for 2.5h in an ice bath. Then, a compound in which C-12 of the arteannuin was connected with an alkoxy group (3.47g, 6 mmol) was added. The reaction solution was stirred for 12 h under 0°C, and then stirred for 24 h under 25°C. Then, the reaction solution was evaporated to dryness under reduced pressure at 60°C. The residue was fully dissolved in water, filtered, concentrated in the filtrate, dropped in isopropanol (100 mL) and filtered. The precipitate was collected and dried for 24 h under vacuum at 50°C, thereby to obtain a conjugate of mono-[3-(diethylenetriamido)-6-deoxy]-α-cyclodextrin and the compound in which C-12 of the arteannuin was connected with an alkoxy group (yield: 56%; solubility: 80 mg/mL).

### Example 12

Mono-[6-(triethylenetetraamine)-6-deoxy]-γ-cyclodextrin (1 mmol) was dissolved in anhydrous dimethylamide (50 mL), and cooled to about 0°C. To this solution, dicyclohexyl carbodiimide (DCC, 1.2 g) was added, and stirred for 24h in an ice bath. Then, a compound in which C-12 of the arteannuin was connected with an alkoxy group (3.47g, 6 mmol) was added. The reaction solution was stirred for 24 h under 10°C, and then stirred for 12 h under 45°C. Then, the reaction solution was evaporated to dryness under reduced pressure at 60°C. The residue was fully dissolved in water and filtered. The filtrate was concentrated, and column chromatography using water as an eluent was performed. The solution obtained from the chromatography was collected and concentrated, then dried for 24 h under vacuum at 50°C, thereby to obtain a conjugate of mono-[6-(triethylenetetraamine)-6-deoxy]-γ-cyclodextrin and the compound in which C-12 of the arteannuin was connected with an alkoxy group (yield: 57%; solubility: 98 mg/mL).

The description of examples above is merely used to help understanding of the method of the present invention and core concept thereof. It is to be noted that, various modifications and improvements thereof will be apparent to those skilled in the art

## Claims

1. An arteannuin-cyclodextrin conjugate, wherein the arteannuin compound is connected via an amide bond formed between a carboxyl group thereof and an amine group of a cyclodextrin modified by the amine group.

2. The arteannuin-cyclodextrin conjugate according to claim 1, wherein the cyclodextrin modified by an amine group has a structure shown in formula I: 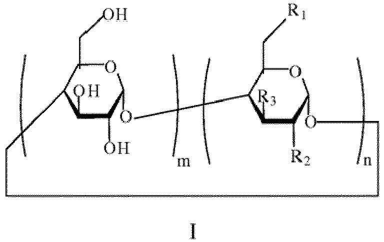
wherein m is 0 to 7; n is 1 to 8, and m+n=any one of 6, 7 and 8;
R₁, R₂ and R₃ represent -OH or -RNH₂, and at least one of R₁, R₂ and R₃ represents -RNH₂;
R represents (CH₂)_{X}, NH(CH₂)_{X}, NH(CH₂)_{X}NH(CH₂)_{X}, CO(CH₂)_{X} or O(CH₂)_{X}, and
x is an integer greater than or equal to 0.

3. The arteannuin-cyclodextrin conjugate according to claim 2, wherein the cyclodextrin modified by an amine group has a structure shown in formula I: 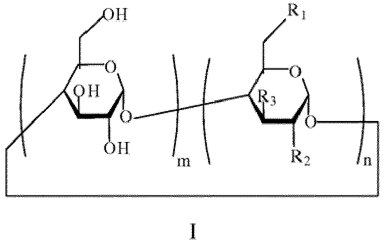
wherein n is 1, and m+n=any one of 6, 7 and 8;
R₁, R₂ or R₃ represents -RNH₂;
R represents (CH₂)_{X}, NH(CH₂)_{X}, NH(CH₂)_{X}NH(CH₂)_{X}, CO(CH₂)_{X} or O(CH₂)_{X}, and
x is 0, 1, 2, 3 or 4.

4. The arteannuin-cyclodextrin conjugate according to claim 2, wherein the cyclodextrin modified by an amine group is selected from the group consisting of mono-[6-(ethanediamine)-6-deoxy]-β-cyclodextrin, mono-[2-(ethanediamine)-6-deoxy]-β-cyclodextrin, mono-[3-(methylamine)-6-deoxy]-α-cyclodextrin and mono- [6-(ethanolamine) -6-deoxy]-γ-cyclodextrin.

5. The arteannuin-cyclodextrin conjugate according to claim 1, wherein the arteannuin compound has the structure shown in formula II: 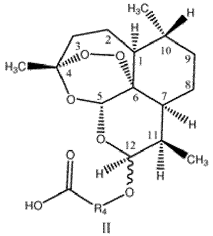 wherein R₄ is (CH₂)_{y}, CO(CH₂)_{y}, C₆H₆(CH₂)_{y} or COC₆H₆(CH₂)_{y}; y is an integer greater than or equal to 0; CO is carbonyl; and C₆H₆ is a benzene ring.

6. The arteannuin-cyclodextrin conjugate according to claim 5, wherein the arteannuin compound is selected from the group consisting of artesunate, a compound in which C-12 of the arteannuin is connected with a phenoxy group, and a compound in which C-12 of the arteannuin is connected with an alkoxy group.

7. A preparation method for the arteannuin-cyclodextrin conjugate according to any one of claims 1 to 6, wherein in the presence of a condensation agent, an amidation reaction between a carboxyl group of the artesunate and an amine group of the cyclodextrin modified by the amine group is carried out in an organic solvent having a strong polarity, thereby to obtain the arteannuin-cyclodextrin conjugate.

8. The preparation method according to claim 7, wherein the condensation agent is a condensation agent of carbodiimides.

9. The preparation method according to claim 7, wherein the condensation agent of carbodiimides is a complex condensation agent of dicyclohexyl carbodiimide and 1-hydroxy benzotriazole.

10. The preparation method according to claim 7, wherein the amidation reaction comprises reacting the cyclodextrin modified by an amine group with excess artesunate under a condition of -10°C to 10°C for 6 to 24 h, and then reacting under a condition of 15°C to 40°C for 6 to 24 h.

11. The preparation method according to claim 7, wherein it further comprises a step of activating the surface hydroxyl group of the cyclodextrin modified by an amine group before the amidation reaction, and said step comprises reacting the cyclodextrin modified by an amine group with the condensation agent under a condition of -10°C to 10°C for 0.5 h to 5 h.

12. The preparation method according to claim 7, wherein it further comprises a step of purifying the arteannuin-cyclodextrin conjugate.

## Patentansprüche

1. Arteannuin-Cyclodextrin-Konjugat, wobei die Arteannuin-Verbindung über eine Amidbindung, die zwischen einer Carboxylgruppe davon und einer Amingruppe eines durch die Amingruppe modifizierten Cyclodextrins gebildet ist, verbunden ist.

2. Arteannuin-Cyclodextrin-Konjugat nach Anspruch 1, wobei das durch eine Amingruppe modifizierte Cyclodextrin eine Struktur aufweist, die in Formel I dargestellt ist: 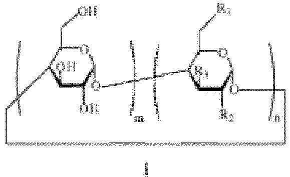
wobei m 0 bis 7 ist; n 1 bis 8 ist, und m + n = ein Beliebiges von 6, 7 und 8;
R₁, R₂ und R₃ -OH oder -RNH₂ darstellen, und mindestens eines von R₁, R₂ und R₃ -RNH₂ darstellt;
R (CH₂)ₓ, NH(CH₂)ₓ, NH(CH₂)ₓNH(CH₂)ₓ, CO(CH₂)ₓ oder O(CH₂)ₓ darstellt und x eine ganze Zahl größer als oder gleich 0 ist.

3. Arteannuin-Cyclodextrin-Konjugat nach Anspruch 2, wobei das durch eine Amingruppe modifizierte Cyclodextrin eine Struktur aufweist, die in Formel I dargestellt ist: 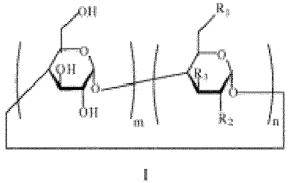
wobei n 1 ist, und m + n = ein Beliebiges von 6, 7 und 8;
R₁, R₂ oder R₃ -RNH₂ darstellt;
R (CH₂)ₓ, NH(CH₂)ₓ, NH(CH₂)ₓNH(CH₂)ₓ, CO(CH₂)ₓ oder O(CH₂)ₓ darstellt und x 0, 1, 2, 3 oder 4 ist.

4. Arteannuin-Cyclodextrin-Konjugat nach Anspruch 2, wobei das durch eine Amingruppe modifizierte Cyclodextrin aus der Gruppe ausgewählt ist, die aus Mono-[6-(ethandiamin)-6-deoxy]-β-Cyclodextrin, Mono-[2-(ethandiamin)-6-deoxy]-β-Cyclodextrin, Mono-[3-(methylamin)-6-deoxy]-α-Cyclodextrin und Mono-[6-(ethanolamin)

5. Arteannuin-Cyclodextrin-Konjugat nach Anspruch 1, wobei die Arteannuin-Verbindung die Struktur aufweist, die in Formel II dargestellt ist: 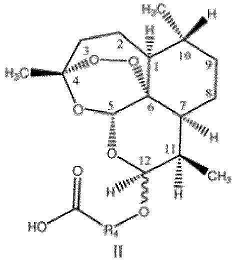 wobei R₄ (CH₂)_{y}, CO(CH₂)_{y}, C₆H₆(CH₂)_{y} oder COC₆H₆(CH₂)_{y} ist; y eine ganze Zahl größer als oder gleich 0 ist; CO Carbonyl ist; und C₆H₆ ein Benzolring ist.

6. Arteannuin-Cyclodextrin-Konjugat nach Anspruch 5, wobei die Arteannuin-Verbindung aus der Gruppe ausgewählt ist, die aus Artesunat, einer Verbindung, bei der C-12 des Arteannuins mit einer Phenoxygruppe verbunden ist, und einer Verbindung, bei der C-12 des Arteannuins mit einer Alkoxygruppe verbunden ist, besteht.

7. Herstellungsverfahren für das Arteannuin-Cyclodextrin-Konjugat nach einem der Ansprüche 1 bis 6, wobei in Gegenwart eines Kondensationsmittels eine Amidierungsreaktion zwischen einer Carboxylgruppe des Artesunats und einer Amingruppe des Cyclodextrins, das durch die Amingruppe modifiziert ist, in einem organischen Lösungsmittel mit einer starken Polarität ausgeführt wird, um dadurch das Arteannuin-Cyclodextrin-Konjugat zu erhalten.

8. Herstellungsverfahren nach Anspruch 7, wobei das Kondensationsmittel ein Kondensationsmittel aus Carbodiimiden ist.

9. Herstellungsverfahren nach Anspruch 7, wobei das Kondensationsmittel aus Carbodiimiden ein komplexes Kondensationsmittel aus Dicyclohexylcarbodiimid und 1-Hydroxybenzotriazol ist.

10. Herstellungsverfahren nach Anspruch 7, wobei die Amidierungsreaktion Folgendes umfasst: Umsetzen des durch eine Amingruppe modifizierten Cyclodextrins mit überschüssigem Artesunat unter einer Bedingung von -10 °C bis 10 °C für 6 bis 24 h und anschließend Umsetzen unter einer Bedingung von 15 °C bis 40 °C für 6 bis 24 h.

11. Herstellungsverfahren nach Anspruch 7, wobei es ferner einen Schritt des Aktivierens der Oberflächenhydroxylgruppe des durch eine Amingruppe modifizierten Cyclodextrins vor der Amidierungsreaktion umfasst, und der Schritt ein Umsetzen des durch eine Amingruppe modifizierten Cyclodextrins mit dem Kondensationsmittel unter einer Bedingung von -10 °C bis 10 °C für 0,5 bis 5 h umfasst.

12. Herstellungsverfahren nach Anspruch 7, wobei es ferner einen Schritt des Reinigens des Arteannuin-Cyclodextrin-Konjugats umfasst.

## Revendications

1. Conjugué artéannuine-cyclodextrine, ledit composé d'artéannuine étant relié par l'intermédiaire d'une liaison amide formée entre un groupe carboxy de celui-ci et un groupe amine d'une cyclodextrine modifiée par le groupe amine.

2. Conjugué artéannuine-cyclodextrine selon la revendication 1, ladite cyclodextrine modifiée par un groupe amine présentant une structure illustrée dans la formule I : 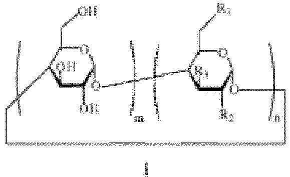
dans laquelle m vaut 0 à 7 ; n vaut 1 à 8, et m+n=un quelconque entier parmi 6, 7 et 8 ;
R₁, R₂ et R₃ représentent un groupe -OH ou -RNH₂, et au moins l'un des groupes R₁, R₂ et R₃ représente un groupe -RNH₂;
R représente un groupe (CH₂)ₓ, NH(CH₂)ₓ, NH(CH₂)ₓNH(CH₂)ₓ, CO(CH₂)ₓ ou O(CH₂)ₓ, et x représente un entier supérieur ou égal à 0.

3. Conjugué artéannuine-cyclodextrine selon la revendication 2, ladite cyclodextrine modifiée par un groupe amine présentant une structure illustrée dans la formule I : 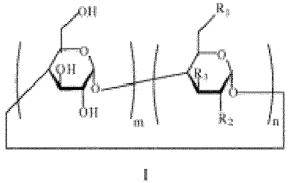
dans laquelle n vaut 1, et m+n=un quelconque entier parmi 6, 7 et 8 ;
R₁, R₂ ou R₃ représente un groupe -RNH₂ ;
R représente un groupe (CH₂)ₓ, NH(CH₂)ₓ, NH(CH₂)ₓNH(CH₂)ₓ, CO(CH₂)ₓ ou O(CH₂)ₓ, et x vaut 0, 1, 2, 3 ou 4.

4. Conjugué artéannuine-cyclodextrine selon la revendication 2, ladite cyclodextrine modifiée par un groupe amine étant choisie dans le groupe constitué par la mono-[6-(éthanediamine)-6-désoxy]-β-cyclodextrine,
la mono-[2-(éthanediamine)-6-désoxy]-β-cyclodextrine,
la mono-[3-(méthylamine)-6-désoxy]-α-cyclodextrine et
la mono-[6-(éthanolamine)-6-désoxy]-γ-cyclodextrine.

5. Conjugué artéannuine-cyclodextrine selon la revendication 1, ledit composé d'artéannuine présentant la structure illustrée dans la formule II : 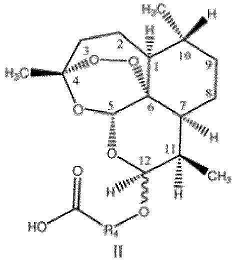 dans laquelle R₄ représente un groupe (CH₂)_{y}, CO(CH₂)_{y}, C₆H₆(CH₂)_{y} ou COC₆H₆(CH₂)_{y} ; y représente un entier supérieur ou égal à 0 ; CO représente un groupe carbonyle ; et C₆H₆ représente un cycle benzénique.

6. Conjugué artéannuine-cyclodextrine selon la revendication 5, ledit composé d'artéannuine étant choisi dans le groupe constitué par l'artésunate, un composé dans lequel l'atome C-12 de l'artéannuine est relié à un groupe phénoxy, et un composé dans lequel l'atome C-12 de l'artéannuine est relié à un groupe alcoxy.

7. Procédé de préparation du conjugué artéannuine-cyclodextrine selon l'une quelconque des revendications 1 à 6, en présence d'un agent de condensation, une réaction d'amidation entre un groupe carboxy de l'artésunate et un groupe amine de la cyclodextrine modifiée par le groupe amine étant effectuée dans un solvant organique présentant une forte polarité, pour obtenir ainsi le conjugué artéannuine-cyclodextrine.

8. Procédé de préparation selon la revendication 7, ledit agent de condensation étant un agent de condensation de carbodiimides.

9. Procédé de préparation selon la revendication 7, ledit agent de condensation de carbodiimides étant un agent de condensation complexe de carbodiimide de dicyclohexyle et de 1-hydroxybenzotriazole.

10. Procédé de préparation selon la revendication 7, ladite réaction d'amidation comprenant la réaction de la cyclodextrine modifiée par un groupe amine avec un excès d'artésunate dans une condition de -10°C à 10°C pendant 6 à 24 h, puis la réaction dans une condition de 15°C à 40°C pendant 6 à 24 h.

11. Procédé de préparation selon la revendication 7, comprenant en outre une étape d'activation du groupe hydroxy de surface de la cyclodextrine modifiée par un groupe amine avant la réaction d'amidation, et ladite étape comprenant la réaction de la cyclodextrine modifiée par un groupe amine avec l'agent de condensation dans une condition de -10°C à 10°C pendant 0,5 h à 5 h.

12. Procédé de préparation selon la revendication 7, comprenant en outre une étape de purification du conjugué artéannuine-cyclodextrine.
